(19) Europäisches Patentamt
European Patent Office
Office européen des brevets

(11) Publication number: **0 058 492**
**A1**

# (12) EUROPEAN PATENT APPLICATION

(21) Application number: 82300548.3

(22) Date of filing: 03.02.82

(51) Int. Cl.³: C 07 D 295/12
C 07 C 87/24, A 61 K 31/04
A 61 K 31/40, A 61 K 31/445
A 61 K 31/535

(30) Priority: 03.02.81 GB 8103281

(43) Date of publication of application:
25.08.82 Bulletin 82/34

(84) Designated Contracting States:
AT BE CH DE FR GB IT LI NL SE

(71) Applicant: GLAXO GROUP LIMITED
Clarges House 6-12 Clarges Street
London W1Y 8DH(GB)

(72) Inventor: Price, Barry John
5 Cowpers Way
Tewin Wood Hertfordshire(GB)

(72) Inventor: Jack, David
6 The Slype Gustard Wood
Wheathampstead Hertfordshire(GB)

(74) Representative: Marchant, James Ian et al,
Elkington and Fife High Holborn House 52/54 High
Holborn
London WC1V 6SH(GB)

## (54) Nitrovinyl derivatives for use in therapy and pharmaceutical compositions containing them.

(57) It has been found that compounds of formula (I)

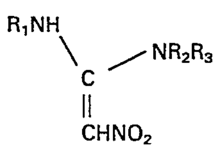

(I)

where $R_1$ represents a $C_{1-6}$ alkyl group, $R_2$ represents a hydrogen atom or a $C_{1-6}$ alkyl group and $R_3$ represents a $C_{1-6}$ alkyl group or $R_2$ and $R_3$ together with the nitrogen atom to which they are attached form a morpholino, pyrrolidino or piperidino group, react with nitrite under physiological conditions to form non-mutagenic derivatives. The invention relates to compounds of formula (I) for use in therapy and to pharmaceutical compositions containing such compounds.

EP 0 058 492 A1

Nitrovinyl Derivatives for Use in Therapy and Pharmaceutical Compositions Containing Them.

The present invention relates to nitrovinyl derivatives for use in therapy, and pharmaceutical compositions containing them.

The presence of nitrite in the stomach is well established. It is derived from foodstuff directly, in which it is used as a preservative, and it is also produced in the body from the nitrate in vegetables and is swallowed with the saliva. It is also known that certain commonly prescribed medicaments and substances resulting from ingestion of food in the stomach react with nitrite under physiological conditions to yield mutagenic and or carcinogenic N-nitrosamines. These N-nitrosation reactions can take place in the stomach.

It is an object of the present invention to prevent or significantly reduce the extent to which such N-nitrosation reactions can occur in the stomach by administering to the patient a compound which will irreversibly react with the nitrosating agent to form a non-mutagenic or non-carcinogenic agent.

The present invention is based on the finding that compounds of formula I

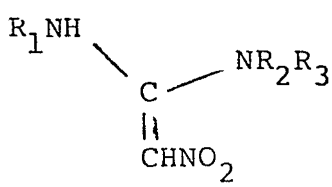

(I)

where $R_1$ represents a $C_{1-6}$ alkyl group, $R_2$ represents a hydrogen atom or a $C_{1-6}$ alkyl group and $R_3$ represents a $C_{1-6}$ alkyl group or $R_2$ and $R_3$ together with the nitrogen atom to which they are attached form a morpholino, pyrrolidino or piperidino group, react with nitrite under physiological conditions to form non-mutagenic

derivatives of formula (II)

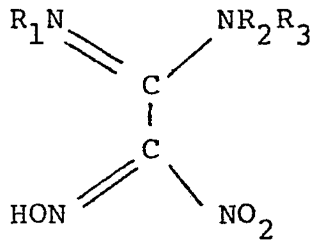

(II)

The present invention provides compounds of formula (I) as defined above for use in a method of therapy practiced on the human or animal body, especially for use in the prevention or reduction of the formation of potentially mutagenic and or carcinogenic N-nitrosamines in the stomach.

The present invention also provides a pharmaceutical composition comprising as active ingredient at least one compound of formula I as defined above together with at least one pharmaceutically acceptable carrier or excipient, and optionally one or more further active ingredients (particularly medicaments containing an N-nitrosatable group).

The term "pharmaceutical composition" as used herein, includes compositions for administration to animals as well as humans, but is not intended to include mere solutions of the compounds of formula I in non-sterile water or a common organic solvent. Pharmaceutical compositions for oral administration are preferred and may take the form of, for example tablets, capsules, powders, solutions, syrups or suspensions prepared by conventional means with acceptable excipients.

The compound of formula (I) will generally be administered at a daily dose level in the range of 50 mg to 2 g more preferably 100 mg to 500 mg and a convenient daily dosage regime would be 1 to 4 doses. The compound will conveniently be formulated in dosage units, each

dosage unit typically containing from 20 mg to 500 mg of the compound of the invention.

The compound of formula (I) may be administered prior to the patient having a meal or taking a medicament containing an N-nitrosatable group. Alternatively the compound of formula (I) may be administered together with a medicament containing an N-nitrosatable group, the compound of formula (I) and the medicament being administered separately or in the same pharmaceutical composition. Examples of medicaments containing an N-nitrosatable group are tetracyclines such as oxytetracycline, and aminopyrine.

In formula (I) preferably $R_1$ represents $C_{1-4}$ alkyl, e.g. methyl or butyl, $R_2$ represents hydrogen or methyl and $R_3$ represents methyl or $NR_2R_3$ represents morpholino, pyrrolidino or piperidino.

Particularly preferred compounds of formula (I) are:

N-methyl-2-nitro-1-(1-pyrrolidinyl)ethenamine,
N-methyl-2-nitro-1-(1-piperidinyl)ethenamine,
N-methyl-1-(4-morpholinyl)-2-nitroethenamine,
N,N,N'-trimethyl-2-nitro-1,1-ethenediamine,
N,N'-dimethyl-2-nitro-1,1-ethenediamine,
N-butyl-2-nitro-1-(1-pyrrolidinyl)ethenamine.

Compounds of formula (I) may be prepared by treating a compound of formula (III) or (IV) in which L is a leaving group, for example an alkylthio group such as methylthio, with an appropriate amine $R_1NH_2$ or $R_2R_3NH$. The reaction is preferably carried out in a solvent such as an alkanol e.g. methanol or ethanol, with heating.

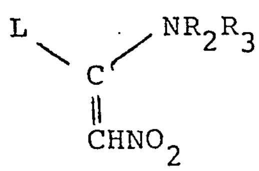

(III)

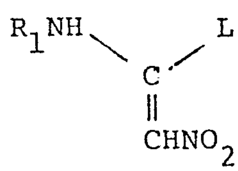

(IV)

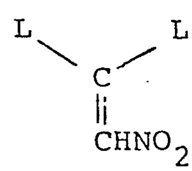

(V)

In a modification of this reaction compounds of formula (I) in which the groups $NR_2R_3$ and $NHR_1$ are the same, may be prepared by treating a compound of formula ( V) in which L is a leaving group as defined above, with an amine $R_1NH_2$. The reaction is conveniently carried out in a solvent, for example an alkanol such as methanol or ethanol and preferably with heating.

The invention is illustrated but not limited by the following examples.

Examples 1 to 6 illustrate the preparation of compounds of formula (I), Examples 7 to 11 describe the reaction of such compounds with sodium nitrite in the presence of acid to form a compound of formula (II) and Example 12 describes pharmaceutical compositions.

EXAMPLE 1

N,N'-Dimethyl-2-nitro-1,1-ethenediamine

Methylamine (100 ml, 35% in ethanol) was added over a ten minute period to a boiling solution of 1,1-bis(methylthio)-2-nitroethene (50g) dissolved in methanol (2 l). After heating at reflux for 30 min, the solvent was evaporated under reduced pressure. Water (100 ml) was added to the concentrated liquors and the crude product isolated by filtration. Recrystallisation from ethanol gave the title compound (15.5 g) as white crystals m.p. 217°C.

EXAMPLE 2

N,N,N'-Trimethyl-2-nitro-1,1-ethenediamine

A solution of N-methyl-1-(methylthio)-2-nitro-ethenamine (14.8 g) in ethanolic N-methyl-methanamine (33%; 100 ml) was heated under reflux for 2 hours. The resulting solutions was evaporated to dryness and recrystallised from ethyl acetate/cyclohexane to give the title compound as a crystalline solid (10.25 g) m.p. 117-119°.

EXAMPLE 3

N-Methyl-1-(4-morpholinyl)-2-nitroethenamine

A mixture of N-methyl-1-(methylthio)-2-nitroethenamine (14.82 g) and morpholine (8.71 g) in acetonitrile (200 ml) was heated at reflux for 30 h and allowed to cool. Removal of the solvent by evaporation gave a solid which was recrystallised from ethyl acetate to give the title compound as a yellow solid: 15.89 g m.p. 133-134.5°.

EXAMPLE 4

N-methyl-2-nitro-1-(1-piperidinyl)ethenamine

A mixture of N-methyl-1-(methylthio)-2-nitroethenamine (14.8 g) and piperidine (8.5 g) in acetonitrile (200 ml) was heated at reflux for twelve hours. The acetonitrile was removed in vacuo to leave a solid which was recrystallised from ethyl acetate/cyclohexane. Column chromatography (Silica 300 g, ethyl acetate) followed by recrystallisation from ethyl acetate gave the title compound (8.2 g) m.p. 115 - 117°.

EXAMPLE 5

N-Methyl-2-nitro-1-(1-pyrrolidinyl)ethenamine

A mixture of N-methyl-1-(methylthio)-2-nitroethenamine (14.82 g) and pyrrolidine (7.82 g) in acetonitrile (200 ml) was heated at reflux for 16h and allowed to cool. The precipitated solid was collected by filtration and recrystallised from ethyl acetate/ethanol. The title compound was collected as pale yellow needles: 15.22 g m.p. 174.5 - 176.5°.

EXAMPLE 6

N-Butyl-2-nitro-1-(1-pyrrolidinyl)ethenamine

(a) N-Butyl-2-(methylthio)-1-nitroethenamine

A solution of 1,1-bis-(methylthio)-2-nitroethene (15 g) in boiling methyl acetate (250 ml) was treated with a solution of n-butylamine (3.3 g) in methyl acetate (50 ml) over 4 hrs. The excess of starting material was removed by filtration, and the filtrate was evaporated. The residue was chromatographed on silica using dichloromethane:methanol (98:2) as eluent to give a solid residue which was crystallised from light petroleum (b.p. 60-80°C) to give the title compound (4.8 g) as off-white crystals m.p. 45-47°.

(b) N-Butyl-2-nitro-1-(1-pyrrolidinyl)ethenamine

A mixture of N-butyl-2-(methylthio)-1-nitroethenamine (4.41 g) and pyrrolidine (1.79 g) in acetonitrile (40 ml) was stirred at room temperature for 20 hrs. The solvent was removed in vacuo and the residue was chromatographed on silica using dichloromethane:methanol (25:1) as eluent, to give a solid. This solid was triturated with pentane to give the title compound (2.66 g) as an off-white solid m.p. 47-49°.

| | | | |
|---|---|---|---|
| Found: | C, 56.3; | H, 9.0; | N, 19.7. |
| $C_{10}H_{19}N_3O_2$ requires: | C, 56.7; | H, 9.4; | N, 20.0%. |

EXAMPLE 7

2-(Hydroxyimino)-N,N-dimethyl-2-nitroethanimidamide

A solution of the compound of Example 1 (2.62 g) and sodium nitrite (1.40 g) in water (50 ml) at 0°C was

treated dropwise with 1N hydrochloric acid (20 ml) over 20 min. An orange solid precipitated and gas was vigorously evolved. The orange solid was filtered off and dried in vacuo to give the <u>title compound</u> m.p. 114°.

<u>EXAMPLE 8</u>

<u>2-(Hydroxyimino)-N,N,N'-trimethyl-2-nitroethanimidamide</u>

The compound of Example 2 (2.9 g) and sodium nitrite (1.38 g, ~~0.02 g~~) in water (50 ml) was stirred at 5°C and 1 N hydrochloric acid (20 ml) was added dropwise over a period of 20 minutes, during the addition the solution turning dark red in colour. The solution was stored at 0°C for 16 hours, the pH of the solution adjusted to pH 12 by the addition of potassium carbonate and the mixture extracted with iso-propanol (3 x 100 ml). The combined extracts were dried ($MgSO_4$), filtered, evaporated to dryness and stored at 0°C for two days. The solid was treated with ethyl acetate (250 ml) and filtered. Recrystallisation of the product from ethanol gave the <u>title compound</u> m.p. 112°.

<u>EXAMPLE 9</u>

<u>4-[2-(Hydroxyimino)-1-(methylimino)-2-nitroethyl]morpholine</u>

Hydrochloric acid (1N, 10.7 ml) was added dropwise to a cooled (5°C) solution of the compound of Example 3 (2.00 g) and sodium nitrite (0.74 g) in water (30 ml). The solution turned deep red and a brown solid was precipitated. The solid was collected by filtration and washed with water (2 x 10 ml) and dried at 20°C in a vacuum pistol to give the <u>title compound</u> (0.71 g) m.p. 111 - 112°.

In a similar manner 1-[2-(hydroxyimino)-1-(methylimino)-nitroethyl]piperidine (0.2 g) m.p. 107 was obtained from N-methyl-2-nitro-1-(1-piperidinyl)etheneamine (1.0 g).

EXAMPLE 10

1-[2-(Hydroxyimino)-1-(methylimino)-2-nitroethyl]pyrrolidine

Hydrochloric acid (1N, 11.7 ml) was added dropwise to a cooled (5°C) solution of the compound of Example 5 (2.00 g) and sodium nitrite (0.81 g) in water (60 ml). A deep red solution resulted which afforded a brown crystalline material. After stirring for 15 min. the title compound (1.2 g) was collected by filtration m.p. 126 - 127°.

EXAMPLE 11

1-[1-(Butylimino)-2-(hydroxyimino)-2-nitroethyl]pyrrolidine

A solution of sodium nitrite (0.28 g) and the product of Example 6 (0.7 g) in water (5 ml) at 5°C was treated with 2N hydrochloric acid (1.75 ml). The resulting precipitate was collected by filtration, washed with water and ether and subsequently dried *in vacuo*, to give the title compound (0.42 g) as an orange solid m.p. 102-103°.

EXAMPLE 12

Pharmaceutical compositions.

(a) Oral Tablets 200 mg

| | mg/tablet |
|---|---|
| Active ingredient | 200.00 |
| Lactose B.P. | 130.00 |
| Starch B.P. | 40.00 |
| Pregelatinised Maize Starch B.P. | 20.00 |
| Sodium Starch Glycolate NF | 8.00 |
| Magnesium Stearate B.P. | 2.00 |
| Compression weight | 400.00 mg |

The active ingredient is sieved through a 250µm sieve and blended with the lactose, starch and pregelatinised starch. The mixed powders are moistened with purified water, granules are made, dried, screened and blended with the Sodium Starch Glycolate and Magnesium Stearate. The lubricated granules are compressed into tablets using 10.0 mm punches. Tablets of other strengths may be prepared by altering the compression weight and using punches to suit.

The tablets may be film coated with suitable film forming materials, e.g. methyl cellulose or hydroxypropyl methyl cellulose using standard techniques. Alternatively the tablets may be sugar coated.

(b) Oral Capsules 200 mg

| | mg/capsule |
|---|---|
| Active ingredient | 200.00 |
| *STA-RX 1500 | 98.5 |
| Magnesium Stearate B.P. | 1.5 |
| Fill weight | 300.00 mg |

* A form of directly compressible starch supplied by Colorcon Ltd., Orpington, Kent.

The active ingredient is seived through a 250μm sieve and blended with the other materials. The mix is filled into No. 2 hard gelatin capsules using a suitable filling machine. Other doses may be prepared by altering the fill weight and if necessary changing the capsule size to suit.

(c) Oral Syrup

| | mg/5 ml dose |
|---|---|
| Active ingredient | 200 |
| Sucrose B.P. | 2750 |
| Glycerine B.P. | 500 |
| Buffer ) | |
| Flavour ) | |
| Colour ) | as required |
| Preservative ) | |
| Distilled Water | 5.00 ml |

The active ingredient, buffer, flavour, colour and preservative are dissolved in some of the water, and the glycerine is added. The remainder of the water is heated to 80°C and the sucrose is dissolved in this and cooled. The two solutions are combined, adjusted to volume and mixed. The syrup produced is clarified by filtration.

The compounds of formula (I) are non-toxic at physiologically useful doses. For example the compound of Example 1 when given to the conscious mouse at doses up to 800 mg/kg orally produced no observable effects.

CLAIMS

1. Compounds of formula (I) for use in a method of therapy practiced on the human or animal body

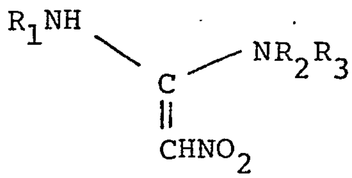

(I)

where $R_1$ represents a $C_{1-6}$ alkyl group, $R_2$ represents a hydrogen atom or a $C_{1-6}$ alkyl group and $R_3$ represents a $C_{1-6}$ alkyl group or $R_2$ and $R_3$ together with the nitrogen atom to which they are attached form a morpholino, pyrrolidino or piperidino group.

2. Compounds as claimed in Claim 1 in which $R_1$ represents $C_{1-4}$ alkyl and either $R_2$ represents hydrogen or methyl and $R_3$ represents methyl or $NR_2R_3$ represents morpholino, pyrrolidino or piperidino.

3. N-Methyl-2-nitro-1-(1-pyrrolidinyl)ethenamine for use in a method of therapy practiced on the human or animal body.

4. N-Methyl-2-nitro-1-(1-piperidinyl)ethenamine for use in a method of therapy practiced on the human or animal body.

5. N-Methyl-1-(4-morpholinyl)-2-nitroethenamine for use in a method of therapy practiced on the human or animal body.

6. N,N,N'-Trimethyl-2-nitro-1,1-ethenediamine or N,N-Dimethyl-2-nitro-1,1-ethenediamine for use in a method of therapy practiced on the human or animal body.

use in a method of therapy practiced on the human or animal body.

7. N-Butyl-2-nitro-1-(1-pyrrolidinyl)ethenamine for use in a method of therapy practiced on the human or animal body.

8. A pharmaceutical composition comprising at least one compound of formula (I) as defined in any of claims 1 to 7 together with at least one pharmaceutically acceptable carrier or excipient.

9. A pharmaceutical composition as claimed in claim 8 also containing a medicament containing an N-nitrosatable group.

10. A pharmaceutical composition as claimed in claim 8 or 9 formulated in dosage units and adapted for oral administration.

0058492

European Patent Office

# EUROPEAN SEARCH REPORT

Application number

EP 82300548.3

| Category | Citation of document with indication, where appropriate, of relevant passages | Relevant to claim | CLASSIFICATION OF THE APPLICATION (Int. Cl. 3) |
|---|---|---|---|
| P,A | GB - A - 2 067 991 (GLAXO) (05-08-1981)<br>* Page 1, formula III, lines 13,14; page 2, lines 19-23 *<br>& BE-A - 886 997 (07-07-1981)<br>& DE-A1-3 100 364 (19-11-1981)<br>& FR-A -2 473 044 (10-07-1981)<br>& NL-A -8 100 068 (03-08-1981)<br>& SE-A -8 100 074 (09-07-1981)<br>-- | 1-4 | C 07 D 295/12<br>C 07 C 87/24<br>A 61 K 31/04<br>A 61 K 31/40<br>A 61 K 31/445<br>A 61 K 31/535 |
| A | DE - A - 2 640 094 (SHELL)<br>* Example 3; claim 2 *<br>---- | 1,6 | |

TECHNICAL FIELDS SEARCHED (Int.Cl. 3)

C 07 D 295/00
C 07 C 87/00
A 61 K 31/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| VIENNA | 06-05-1982 | BRUS |